# EUROPEAN PATENT APPLICATION

(11) **EP 2 574 348 A1**
(43) Date of publication of application: **03.04.2013**
(21) Application number: 11183196.2
(22) Date of filing: 28.09.2011
(51) Int. Cl.: A61L 2/232, A01N 25/34, E05B 1/00, A01N 25/24

(54) **Disinfecting foil for covering handles, head restraint and cushions' drape**

(71) Applicant: FP PROGETTI ITALIA S.r.L. Unipersonale, 39042 Bressanone (Bolzano) (IT)
(72) Inventor: Lazzari, Massimiliano, 24060 Endine Gaiano (Bergamo) (IT)
(74) Representative: Lunati & Mazzoni S.r.L.

(57) **Abstract**

It is provided a hygienic covering device (10) for handles (30) and the like, comprising: a substrate (11) consisting of a membrane of nonwoven textile material including an outer surface (11a) adapted to be grasped by a user and a back face (11b) opposite to said outer surface (11 a), a bactericidal substance impregnating the substrate (11) at least on the outer surface (11a), an adhesive layer (12) consisting of adhesive of the releasable type disposed on part of the back face (11b), wherein the substrate (11) comprises a side portion (13) devoid of the adhesive layer (12), (12), extending over the entirety of one side of the substrate (11) and adapted to constitute a liftable grip.

## Description

The present invention relates to a hygienic covering device for handles in general, such as small handles for trays, handgrips for trolleys, headrests, covercloths for cushions/pillows and similar goods (hereinafter generally referred to as "hygienic device") of the type pointed out in the preamble of the independent claims.

It is generally known that microbes and bacteria or other elements causing skin infections are usually present on surfaces and objects designed to come into contact with the hands or other unprotected parts of the human body, as it happens for instance for handgrips of trolleys in big shopping centres, sports equipment, refectories, headrests on aeroplanes, ships. trains, public transport means by road or in other applications in which the very bad hygienic and sanitary conditions can be the origin of serious infections, both by immediate contagion and in cross form.

Infections due to cross contamination represent a serious problem for public health, because bacteria, microbes, moulds and parasites easily spread both in highly crowded places, due for example to the increase in business and trade and the growing resorting to public catering, and in public places where insufficient care is given to hygiene.

High temperature, presence of oxygen in public places where forced-air conditioning systems are often present, and weather are all determinant factors contributing in quick proliferation of micro-organisms adapted to cause diseases and dangerous infections; generally, hands and surfaces in very bad hygienic conditions are the main vehicle for diffusion and contamination from microbes and bacteria.

It is therefore apparent that it is important to act in such a manner as to reduce all risks of contamination, by adopting solutions capable of limiting every direct contact with infected objects and/or surfaces at best.

In this regard, many attempts have been made by adopting sprays and disinfectant or sanitising liquids for preventive or belated disinfecting, as well as through use of covering elements adapted to interpose a mere physical barrier between the hands and the contaminated surface.

In general, use of a sheet or a sheath of plastic material, fabric, paperboard or other material is suggested.

Other known devices are impregnated with sterilising substances.

The known art mentioned above has some important drawbacks.

In fact, use of known devices is often uncomfortable and complicated.

In addition, known devices may not be able to constitute a sufficient barrier against microbes and bacteria.

On the contrary, the devices impregnated with sterilising substances are often offensive for the hands or the user's skin.

Under this situation, the technical task underlying the present invention is to conceive a "hygienic device" capable of substantially obviating the mentioned drawbacks.

The technical task mentioned and the aims specified are achieved by a "hygienic device" as claimed in the appended independent claims.

Preferred embodiments are highlighted in the sub-claims.

The features and advantages of the invention are hereinafter clarified by the detailed description of preferred embodiments of the invention, with reference to the accompanying drawings, in which:
**Fig. 1** is a view of a hygienic covering device for handles and the like according to the invention;
**Fig. 2** is a section taken along line II-II in Fig. 1;
**Fig. 3** shows the device according to the invention in roll;
**Fig. 4** is a flow diagram of the production process and the method of obtaining the material of the device; and
**Fig. 5** shows use of the device according to the invention.

With reference to the drawings, the "hygienic device" according to the invention is generally identified by reference numeral **1.**

The hygienic covering device, generally denoted at **10,** comprises a substrate **11** consisting of a membrane of nonwoven textile material, a nonwoven fabric for example, obtained by crossing a plurality of fibres disposed in layers and joined together by a needle punching process, or caused to mutually adhere by a suitable glue or bonding agent.

The substrate 11 is in the form of a veil the thickness and sizes of which can vary depending on the provided application; by way of example, thickness of the substrate 11 can be such that its weight can vary between 20 and 50 g/m², preferably between 25 and 35 g/m², while the other sizes are exclusively in relation to the field of use, as illustrated hereinafter.

While in principle the substrate 11 can consist of any material suitable for the stated use, planning and selection of a substrate of nonwoven textile material is appropriate due to a good ratio between tearing resistance and antibacterial capacity, intended as the capacity to be impregnated with a metered amount of a bactericidal composition.

While in the medical, paramedical, aesthetic and food field the nonwoven fabric has found since long a specific position and use, in the present invention it is proposed as an appropriate support for being pre-impregnated with a solution of a bactericidal composition and for being subsequently submitted to a drying step for complete evaporation of the solvent, leaving an active dry residue on the outer surface **11a** and in the fabric layer 11, which residue is able to softly reduce the bacterial amount present on surfaces to be covered or on that part of the user's body it comes in contact with.

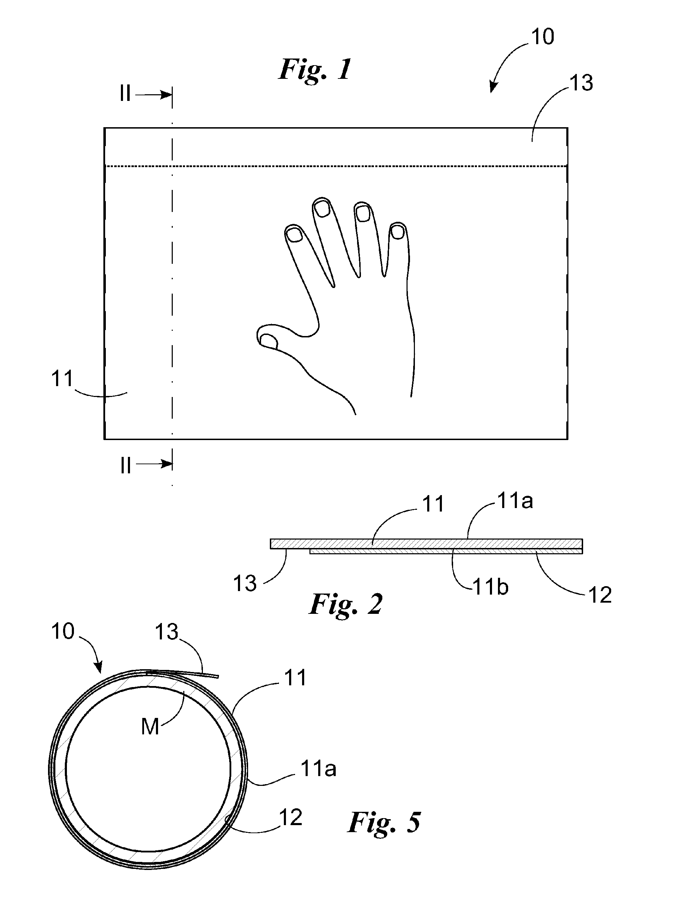

Still with reference to Figs. 1 and 2, device 10, on the back face **11 b** opposite to the outer surface 11a, is partly provided with an adhesive layer **12** consisting of an adhesive substance of the releasable type, a resin-based adhesive for example that can also consist of a biodegradable acrylic resin, in the same way as the substrate 11 fibres.

The adhesive layer 12 can be spread by a spreading process for example, so as to cover the back surface of substrate 11 almost completely, except for two portions or marginal lifting parts 13 along one or more peripheral edges.

In the example shown, the adhesive layer 12 is spread over the whole back surface of substrate 11, leaving a peripheral side portion **13** without glue, for lifting of the flap and separation of the sheet of material from the surface to which it has been made to adhere, when it is no longer necessary.

The side portion 13 extends over the entirety of one side of substrate 11 so that it can constitute a liftable grip, as shown in Fig. 5. In the examples shown, substrate 11 is of rectangular shape and portion 13 is a band extending over the entirety of one side. However the shape could be different and the side portion could consist of a pointed or triangular-shaped side or a tape or string discontinuous with the rest of the substrate but at all events adapted to be manually grasped because it is not laterally constrained by the adhesive layer 12.

Finally, the side portion 13 preferably takes up a part of the substrate membrane in a direction perpendicular to the direction of the side it takes up, that is less than 10% of the overall length of the substrate in the same direction. As an alternative to the above, it is possible for the adhesive layer 12 to be distributed in strips, leaving at least the side portion 13 of substrate 11 free over the whole length thereof, which is useful to make lifting and separation easier.

If the adhesive layer 12 is spread over the whole back surface of substrate 11, except the side portion 13, and subsequent calendering steps are carried out to make the spreading step uniform, the continuous adhesive film penetrates into the fibres of the substrate 11 becoming part thereof; in this manner it constitutes a physical barrier to passage of microbes and bacteria in both directions, i.e. from the covered surface towards the user and in the opposite direction.

The main feature of the present invention consists in pre-impregnating the substrate 11 with a solution of a bactericidal composition or substance in a measured amount and in submitting the pre-impregnated substrate before the adhesive layer 12 is spread thereon, to a heating and drying step causing evaporation of the solvent.

As the bactericidal substance, any composition suitable for the purpose is used, which substance must be able to sanitise and clean, while at the same time not being offensive for the skin of the hands or the body portion in contact with the device. For example, chlorhexidine is preferred, which is normally used in skin disinfection in the form of an impregnated sponge for preparation of the surgeons' hands before every operation or orally used in smaller percentages in the form of mouthwash.

From tests carried out on a series of samples it has been ascertained that, by operating with mass amounts of dry residue of chlorhexidine in substrate 11 smaller than 0.5% relative to the substrate mass, good results were obtained for sanitisation and as a valid hygienic aid against cross contamination in many application sectors.

Contrary to normal use of chlorhexidine in liquid solution, or other appropriate composition of similar function (still in liquid solution), use of a substrate pretreated with an antibacterial agent and dried is very advantageous because the residue does not involve alteration of the mucous membranes or presence of residual product on the skin.

As previously said, as an alternative to chlorhexidine, any other product or disinfectant having antibacterial or sanitising properties, in particular skin-compatible can be used; it is also to be pointed out that the drying process of the bactericidal substance also allows use of same by those who have open wounds on the hands or body parts that have to come into contact with the hygienic covering material according to the present invention.

Device 10 can be made in any manner; it can be in the form of individual sheets packaged in a suitable envelope or in the form of roll material that can be picked up from a suitable dispenser studied and produced by the Parent Company.

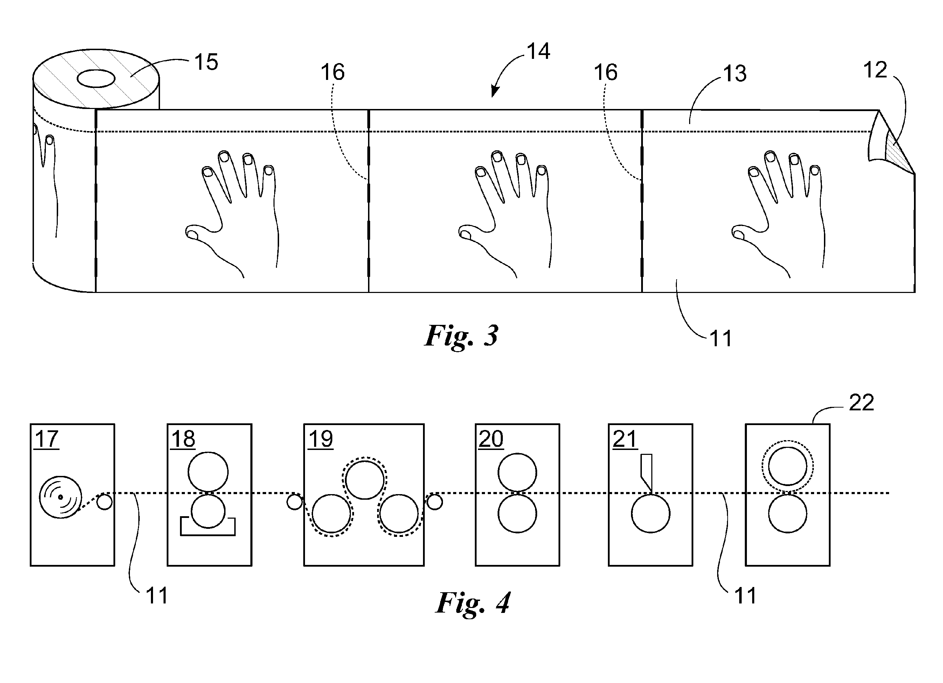

A material in roll is shown by way of example in Fig. 3, while the block diagram in Fig. 4 describes a possible production process.

According to the embodiment in Fig. 3, in which the same reference numerals as in the preceding figures have been used for denoting similar or equivalent parts, the product is in the form of a ribbon **14** that is unwound from a roll **15;** the ribbon 14 comprises a plurality of weakened lines **16** obtained by a pre-cut carried out using a hollow punch, which lines extend transversely over the whole width of ribbon 14 and divide it into individual sheets of predetermined length, depending on use requirements.

As an alternative to the example in Fig. 3, ribbon 14 could be devoid of the tear-off lines 16; in this case the material in roll 15 could be placed in a dispenser provided with a cutting device or previously packaged in predetermined sizes. A possible production process will be now described with reference to the flow diagram in Fig. 4 wherein the different steps have been symbolically represented by respective blocks; the production process can take place both without interruption along the same production line starting from the step of forming the nonwoven fabric to the final step of cutting it and winding the individual strips of material into rolls (continuous process), and in separated steps or groups of steps (discontinuous process).

In general, after formation of the substrate 11 of nonwoven fabric in the form of a mat wound up into a roll, the substrate is unrolled, step **17,** and conveyed to step **18** where the substrate 11 is impregnated with a metered amount of a bactericidal composition, as previously said.

After step 18 for impregnating the substrate 11 of nonwoven fabric with the bactericidal composition, said substrate is directed to a heating and drying step **19** carried out by means of a calender system, and the substrate of nonwoven fabric is heated to a temperature suitable to cause full evaporation of the solvent, leaving the substrate 11 impregnated with a dry residue of bactericidal substance.

When step 19 for drying the bactericidal composition has been completed, if printing is required, the substrate 11 is sent to a printing step **20** during which any indication, information or image adapted to show the mode of use or advertising messages is printed on the front or outer surface 11 a thereof; printing of the substrate 11 can be carried out using any type of printing element, preferably ink.

When printing of the substrate 11 has been completed, a step **21** of laying an adhesive of the releasable type, by spreading, is carried out, preferably an adhesive having an acrylic base being used.

At the end of the adhesive spreading and drying step the substrate 11 is sent to step **22** for cutting the material into ribbons and/or carrying out the transverse tear-off weakening lines 16, as previously said, then packaging is finally accomplished.

Use of the device 1, previously described in terms of structure is hereinafter described.

Device 10 can be used for handles **30** present in public places. In particular, these handles 30 are: handgrips for shopping trolleys in supermarkets or shopping centres, handgrips of luggage trolleys in airports and stations, small handles of trays for fast-food and the like, handgrips for sports equipment, door handles, sanitary and aesthetic equipment and headrests, covercloths for pillows/cushions and others.

Devices 10 are therefore positioned inside an appropriate dispenser in the vicinity of the objects including said handles 30 to be coated. The dispenser suitably comprises a roll 15 and cutting mechanisms for the devices 10 that can be of the manual or automatic type.

In particular, it is important for the user to only grasp the device 10 he/she wants to use.

Therefore the user grasps the device 10 along the outer surface 11a of the substrate and wraps said device 10 around handle 30.

Due to the presence of the adhesive layer 12, the device adheres to the handle except for the side portion 13, as shown in Fig. 5.

The user therefore uses handle 30 as he/she habitually does but he/she comes into contact with a surface consisting of the outer surface 11a of the device 10 that is not only clean because nobody else has touched it, but also active, due to the presence of the bactericidal substance.

At the same time, since the bactericidal substance is not a sterilising or germicidal substance, it does not cause offensive actions on the user's skin.

When the user has finished use of the device 10, because he/she does no longer need the trolley including handle 30 for example, he/she can easily detach the device 10 from the handle grasping the side portion 13 thereof and unwinding the device 10 itself.

Disposal of device 10 takes place in a suitable container.

The invention has important advantages.

In particular, device 10 is of very practical and simple use.

These advantages are due to the selection of bactericidal substances that are not sterilising substances and also to the particular application and evaporation process described above.

A further advantage resides in use of a substrate of nonwoven fabric, the tearing and manipulation resistance of which is greater as compared with any other paper product.

In addition, the nonwoven fabric impregnated with the bactericidal composition, unlike paper material, keeps quite unchanged the features of resistance to manipulation also after being impregnated.

Use of the "hygienic device" 10 is representative and explanatory but it does not constitute a constraint for use and application of the other "hygienic devices" being the object of the present document.

## Claims

1. A hygienic covering device (10) for handles (30) and the like such as headrests and covercloths for cushions, comprising:
- a substrate (11) consisting of a membrane including an outer surface (11a) adapted to be grasped by a user and a back face (11 b) opposite to said outer surface (11a),
- a bactericidal substance impregnating said substrate (11) at least on said outer surface (11a),
- an adhesive layer (12) consisting of adhesive of the releasable type disposed on part of said back face (11 b),
**characterised in that:**
- said substrate (11) is a membrane of nonwoven textile material comprising a side portion (13) devoid of said adhesive layer (12), (12), extending over the entirety of one side of said substrate (11) and adapted to constitute a liftable grip.

2. A device as claimed in claim 1, wherein said side portion (13) preferably takes up a portion of substrate (11) in a direction perpendicular to the direction of the side it takes up, that is less than 10% of the overall length of said substrate (11) in the same direction.

3. A device as claimed in one or more of the preceding claims, wherein said bactericidal substance is chlorhexidine or other similar substance.

4. A device as claimed in claim 3, wherein said chlorhexidine is in mass amounts of dry residue in the substrate (11) smaller than 0.3% relative to the substrate mass.

5. A process for producing a hygienic covering device (10) for handles (30) and the like such as headrests and covercloths for cushions, said hygienic covering device (10) comprising:
- a substrate (11) consisting of a membrane of nonwoven textile material including an outer surface (11a) adapted to be grasped by a user and a back face (11 b) opposite to said outer surface (11 a),
said process being **characterised in that** it comprises:
- an impregnating step (18) in which the substrate (11) is impregnated with a metered amount of a bactericidal composition,
- a heating and drying step (19), in which the substrate (11) is heated to a temperature adapted to cause full evaporation of the solvent,
- a step (21) of laying an adhesive layer (12) by spreading, which layer consists of adhesive of the releasable type disposed on part of said back face (11 b).

6. A process as claimed in claim 5, wherein said bactericidal substance is chlorhexidine or other similar substance.
